# EUROPÄISCHE PATENTSCHRIFT

(11) **EP 0 677 510 B1**
(45) Veröffentlichungstag und Bekanntmachung des Hinweises auf die Patenterteilung: **28.01.1998**
(21) Anmeldenummer: 95105255.4
(22) Anmeldetag: 07.04.1995
(51) Int. Cl.: C07C 309/51, C07C 275/34, C07C 275/40

(54) **Hydroxyphenylharnstoffe**
Hydroxyphenylureas
Hydroxyphénylurées

(30) Priorität: 16.04.1994 DE 4413265
(43) Veröffentlichungstag der Anmeldung: 18.10.1995
(73) Patentinhaber: BASF AKTIENGESELLSCHAFT, 67056 Ludwigshafen (DE)
(72) Erfinder: Marschner, Claus, Dr., D-67346 Speyer (DE); Kessel, Knut, Dr., D-68161 Mannheim (DE); Patsch, Manfred, Dr., D-67157 Wachenheim (DE)

(56) Entgegenhaltungen:
- EP-A- 0 592 980
- JOURNAL FÜR PRAKTISCHE CHEMIE, Bd. 48, 1893 LEIPZIG, DE, Seiten 425-446, R. SCHMITT: 'Über die Einwirkung con Chlorkohlenoxyd auf Pikraminsäure'

## Beschreibung

Die vorliegende Erfindung betrifft Harnstoffe der Formel I 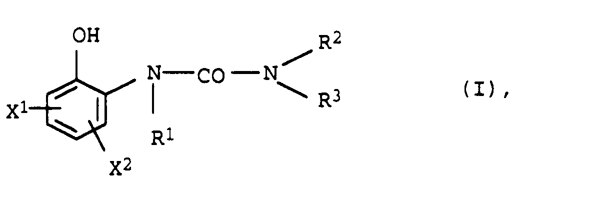 in der
- X¹: Nitro oder Amino,
- X²: Nitro, Amino, Hydroxysulfonyl, Sulfamoyl, C₁-C₄-Mono- oder Dialkylsulfamoyl oder einen Rest der Formel -S(O)ₙ-Y, worin n für 0 oder 2 und Y für Vinyl oder einen Rest der Formel C₂H₄-Q stehen, in dem Q die Bedeutung von Hydroxy oder einer unter alkalischen Reaktionsbedingungen abspaltbaren Gruppe besitzt,
- R¹: Wasserstoff, C₁-C₆-Alkyl, das gegebenenfalls substituiert ist und durch 1 oder 2 Sauerstoffatome in Etherfunktion oder Imino- oder C₁-C₄-Alkyliminogruppen unterbrochen sein kann, oder C₃-C₄-Alkenyl,
- R² und R³: unabhängig voneinander jeweils C₁-C₆-Alkyl, das gegebenenfalls substituiert ist und durch 1 oder 2 Sauerstoffatome in Etherfunktion oder Imino- oder C₁-C₄-Alkyliminogruppen unterbrochen sein kann, oder gegebenenfalls substituiertes Phenyl oder R² auch Wasserstoff oder einen Rest der Formel 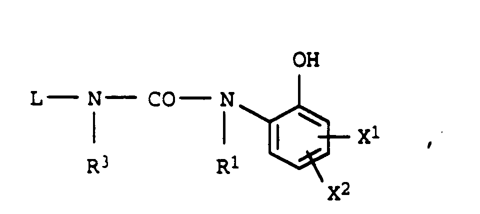 worin L für C₂-C₈-Alkylen oder Phenylen steht und R¹, R³, X¹ und X² jeweils die obengenannte Bedeutung besitzen, oder R² und R³ zusammen auch einen Rest der Formel (-C₂H₄)₂N-R¹, worin R¹ die obengenannte Bedeutung besitzt, bedeuten,
mit der Maßgabe, daß
a) Verbindungen der Formel 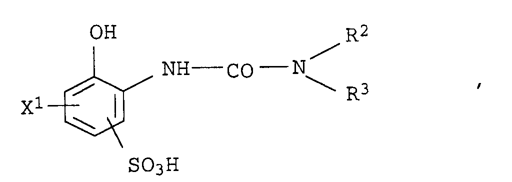 in der X¹ Nitro oder Amino und einer oder beide Reste R² und R³ C₁-C₆-Alkyl, das durch den Rest -S(O)ₙ-Y, in dem n und Y jeweils die obengenannte Bedeutung besitzen, substituiert und gegebenenfalls durch 1 oder 2 Sauerstoffe in Etherfunktion oder Imino- oder C₁-C₄-Alkyliminogruppen unterbrochen ist, bedeuten, und
b) Verbindungen der Formel

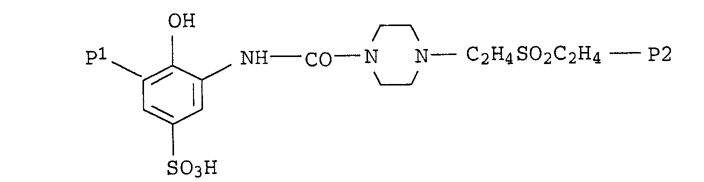

| P¹ | P² |
|---|---|
| NO₂ | OH |
| NH₂ | OH |
| NH₂ | OSO₃H |

und
c) Die Verbindung der Formel 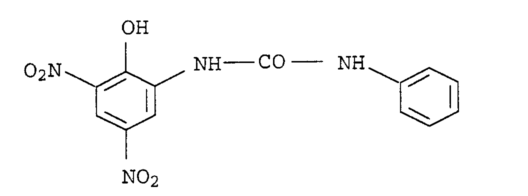 ausgenommen sind, sowie ein Verfahren zu ihrer Herstellung.

Aus der älteren Patentanmeldung EP-A-592 980 sind solche Harnstoffe bekannt, die von der obengenannten Formel I ausgenommen sind.

Weiterhin sind in Comptes rendus de l'Académie bulgare des Sciences, Band 41, Seiten 113 bis 116, 1988, Hydroxyphenylharnstoffe beschrieben, die eine Nitrogruppe im Phenylring aufweisen.

Aufgabe der vorliegenden Erfindung war es, neue Hydroxyphenylharnstoffe bereitzustellen, die sich vorteilhaft zur Herstellung von Metallkomplexfarbstoffen eignen.

Ferner ist in J. für Prakt. Chemie, Bd. 48, 1893, Seiten 425-446 N-2-Hydroxy-3,5-dinitrophenyl-N-phenyl-harnstoff beschrieben.

Alle in der oben genannten Formel auftretenden Alkyl-, Alkenyl- und Alkylengruppen können sowohl geradkettig als auch verzweigt sein.

Wenn in der obengenannten Formel substituierte Alkylgruppen auftreten, so können als Substituenten, z.B. Hydroxy, Amino, Cyano, Carboxyl, Hydroxysulfonyl, Sulfato, Carbamoyl, C₁-C₄-Mono- oder Dialkylcarbamoyl, Piperazino, N-(C₁-C₄-Alkyl)piperazino, Thiomorpholino-S,S-dioxid oder ein Rest der Formel -S(O)ₙ-Y, worin n und Y jeweils die obengenannte Bedeutung besitzen, in Betracht kommen. Die Alkylgruppen weisen in der Regel dann 1 oder 2 gleiche oder verschiedene Substituenten auf.

Wenn in der obengenannten Formel substituierte Phenylgruppen auftreten, so können als Substituenten z.B. C₁-C₄-Alkyl, C₁-C₄-Alkoxy, Halogen, Nitro, Amino, C₁-C₄-Mono- oder Dialkylamino, C₁-C₄-Alkanoylamino, Benzylamino, Carboxyl, Carbamoyl, C₁-C₄-Mono- oder Dialkylcarbamoyl, Hydroxysulfonyl oder ein Rest der Formel -S(O)ₙ-Y, worin n und Y jeweils die obengenannte Bedeutung besitzen, in Betracht kommen. Die Phenylgruppen weisen in der Regel dann 1 bis 3 gleiche oder verschiedene Substituenten auf.

Der Rest Q steht für Hydroxy oder eine unter alkalischen Reaktionsbedingungen abspaltbare Gruppe. Solche Gruppen sind z.B. Chlor, Brom, C₁-C₄-Alkylsulfonyl, Phenylsulfonyl, OSO₃H, SSO₃H, OP(O)(OH)₂, C₁-C₄-Alkylsulfonyloxy, gegebenenfalls substituiertes Phenylsulfonyloxy, C₁-C₄-Alkanoyloxy, C₁-C₄-Dialkylamino oder ein Rest der Formel 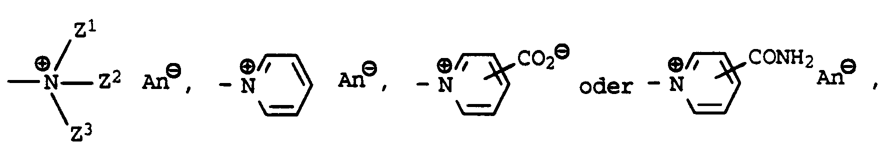 wobei Z¹, Z² und Z³ unabhängig voneinander jeweils die Bedeutung von C₁-C₄-Alkyl oder Benzyl und An^{⊖} jeweils die Bedeutung eines Äquivalents eines Anions besitzen. Als Anionen können dabei z.B. Fluorid, Chlorid, Bromid, Iodid, Mono-, Di- oder Trichloracetat, Methansulfonat, Benzolsulfonat oder 2- oder 4-Methylbenzolsulfonat in Betracht kommen.

Reste R¹, R², R³, Z¹, Z² und Z³ sind z.B. Methyl, Ethyl, Propyl, Isopropyl, Butyl, Isobutyl, sec-Butyl oder tert-Butyl.

Reste R¹, R² und R³ sind weiterhin z.B. Pentyl, Isopentyl, Neopentyl, tert-Pentyl, Hexyl, 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 4-Methoxybutyl, 2- oder 4-Ethoxybutyl, 3,6-Dioxaheptyl, 3,6-Dioxaoctyl, 3-Azapentyl, 3-Methyl-3-azapentyl, 2-Hydroxyethyl, 2- oder 3-Hydroxypropyl, 2- oder 4-Hydroxybutyl, 5-Hydroxypentyl, 6-Hydroxyhexyl, 2-Aminoethyl, 2- oder 3-Aminopropyl, 2- oder 4-Aminobutyl, 5-Aminopentyl, 6-Aminohexyl, Cyanomethyl, 2-Cyanoethyl, 2- oder 3-Cyanopropyl, 2- oder 4-Cyanobutyl, 5-Cyanopentyl, 6-Cyanohexyl, Carbonylmethyl, 2-Carboxyethyl, 2- oder 3-Carboxypropyl, 2- oder 4-Carboxybutyl, 5-Carboxypentyl, 6-Carboxyhexyl, 2-Hydroxysulfonylethyl, 2- oder 3-Hydroxysulfonylpropyl, 2- oder 4-Hydroxysulfonylbutyl, 5-Hydroxysulfonylpentyl, 6-Hydroxysulfonylhexyl, 2-Sulfatoethyl, 2- oder 3-Sulfatopropyl, 2- oder 4-Sulfatobutyl, 5-Sulfatopentyl, 6-Sulfatohexyl, Carbamoylmethyl, 2-Carbamoylethyl, 2- oder 3-Carbamoylpropyl, 2- oder 4-Carbamoylbutyl, 5-Carbamoylpentyl, 6-Carbamoylhexyl, Mono- oder Dimethylcarbamoylmethyl, Mono- oder Diethylcarbamoylmethyl, 2-(Mono- oder Dimethylcarbamoyl)ethyl, 2-(Mono- oder Diethylcarbamoyl)ethyl, 2- oder 3-(Mono- oder Dimethylcarbamoyl)propyl, 2- oder 3-(Mono- oder Diethylcarbamoyl)propyl, 2- oder 4-(Mono- oder Dimethylcarbamoyl)butyl, 2- oder 4-(Mono- oder Diethylcarbamoyl)butyl, 2-(Piperazin-1-yl)ethyl, 2- oder 3-(Piperazin-1-yl)propyl, 2- oder 4-(Piperazin-1-yl)butyl, 2-(4-Methylpiperazin-1-yl)ethyl, 2- oder 3-(4-Methylpiperazin-1-yl)propyl, 2- oder 4-(4-Methylpiperazin-1-yl)butyl, 2-(Thiomorpholin-S,S-dioxid-4-yl)ethyl, 2- oder 3-(Thiomorpholin-S,S-dioxid-4-yl)propyl, 2- oder 4-(Thiomorpholin-S,S-dioxid-4-yl)butyl oder ein Rest der Formel -(CH₂)₂-S-Y, -(CH₂)₂-SO₂-Y, -(CH₂)₃-S-Y, -CH₂-CH(CH₃)-S-Y, -(CH₂)₃-SO₂-Y, -CH₂-CH(CH₃)-SO₂-Y, -(CH₂)₄-S-Y, (CH₂)₄-SO₂-Y, (CH₂)₂O(CH₂)₂-S-Y, (CH₂)₂O(CH₂)₂-SO₂-Y, (CH₂)₂NH(CH₂)₂-S-Y, (CH₂)₂NH(CH₂)₂-SO₂-Y, 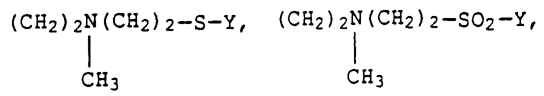 (CH₂)₂O(CH₂)₂O(CH₂)₂-S-Y, (CH₂)₂O(CH₂)₂O(CH₂)₂-SO₂-Y, (CH₂)₂NH(CH₂)₂NH₂(CH₂)₂-S-Y, (CH₂)₂NH(CH₂)₂NH(CH₂)₂-SO₂-Y, 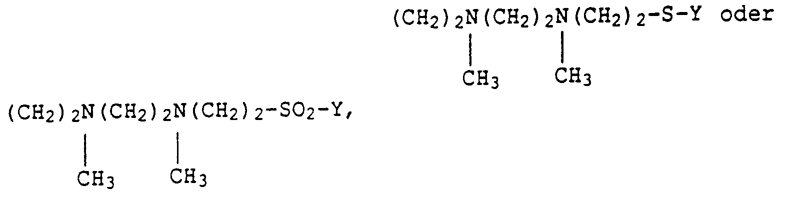 worin Y jeweils die obengenannte Bedeutung besitzt.

Reste R¹ sind weiterhin z.B. Allyl oder Methallyl.

Reste R² und R³ sind weiterhin z.B. Phenyl, 2-, 3- oder 4-Methylphenyl, 2,4-Dimethylphenyl, 2-, 3- oder 4-Methoxyphenyl, 2-, 3- oder 4-Chlorphenyl, 2-, 3- oder 4-Aminophenyl, 2-, 3- oder 4-(Mono- oder Dimethylamino)phenyl, 2-, 3- oder 4-(Mono- oder Diethylamino)phenyl, 2-, 3- oder 4-Formylaminophenyl, 2-, 3- oder 4-Acetylaminophenyl, 2-, 3- oder 4-Propionylaminophenyl, 2-, 3- oder 4-Butyrylaminophenyl, 2-, 3- oder 4-Isobutyrylaminophenyl, 2-, 3- oder 4-carboxyphenyl, 2-, 3- oder 4-Carbamoylphenyl, 2-, 3- oder 4-(Mono- oder Dimethylcarbamoyl)phenyl, 2-, 3- oder 4-(Mono- oder Diethylcarbamoylphenyl), 2-, 3- oder 4-Hydroxysulfonylphenyl oder ein Rest der Formel 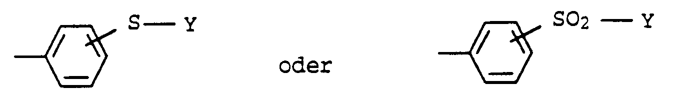 worin Y jeweils die oben genannte Bedeutung besitzt.

Reste X² sind z.B. Mono- oder Dimethylsulfamoyl, Mono- oder Diethylsulfamoyl, Mono- oder Dipropylsulfamoyl, Mono- oder Diisopropylsulfamoyl, Mono- oder Dibutylsulfamoyl oder N-Methyl-N-ethylsulfamoyl.

Reste L sind z.B. (CH₂)₂, (CH₂)₃, (CH₂)₄, (CH₂)₅, (CH₂)₆, (CH₂)₇, (CH₂)₈, CH(CH₃)CH₂, CH(CH₃)CH(CH₃) oder 1,2-, 1,3- oder 1,4-Phenylen.

Bevorzugt sind Harnstoffe der Formel I, in der
- X²: Nitro, Amino, Hydroxysulfonyl oder einen Rest der Formel -SO₂-Y bedeutet, worin Y die obengenannte Bedeutung besitzt, wobei Hydroxysulfonyl oder ein Rest der Formel -SO₂Y besonders zu nennen sind.

Weiterhin bevorzugt sind Harnstoffe der Formel I, in der
- R¹: Wasserstoff oder gegebenenfalls durch Hydroxysulfonyl, Hydroxy oder Sulfato substituiertes C₁-C₆-Alkyl bedeutet, wobei Wasserstoff besonders zu nennen ist.

Weiterhin bevorzugt sind Harnstoffe der Formel I, in der
- R²: Wasserstoff oder C₁-C₆-Alkyl,insbesondere Wasserstoff, und
- R³: C₁-C₆-Alkyl, das gegebenenfalls durch Amino, Hydroxy, Hydroxysulfonyl, Sulfato oder einen Rest der Formel -SO₂-Y, worin Y die obengenannte Bedeutung besitzt, substituiert ist und durch ein Sauerstoffatom in Etherfunktion oder eine Iminogruppe unterbrochen ist, oder Phenyl, das gegebenenfalls durch Amino, C₁-C₄-Monoalkylamino, C₁-C₄-Alkanoylamino, Benzoylamino, Carboxyl, Hydroxysulfonyl oder einen Rest der Formel -SO₂-Y, worin Y die obengenannte Bedeutung besitzt, substituiert ist, oder R² und R³ zusammen einen Rest der Formel (-C₂H₄)₂N-R¹, worin R¹ die obengenannte Bedeutung besitzt, bedeuten.

Weiterhin bevorzugt sind Harnstoffe der Formel I, in der
- L: C₂-C₄-Alkylen oder Phenylen bedeutet.

Die Harnstoffe der Formel I können nach an sich bekannten Methoden, wie sie z.B. in der EP-A-592 980 beschrieben sind, erhalten werden. So kann man z.B. ein Benzoxazolon der Formel II 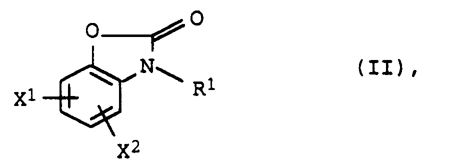 in der X¹, X² und R¹ jeweils die obengenannte Bedeutung besitzen, mit einem Amin der Formel III 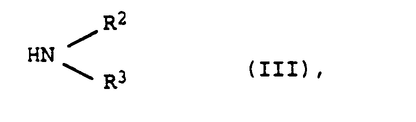 in der R² und R³ jeweils die obengenannte Bedeutung besitzen, umsetzen.

Die neuen Harnstoffe der Formel I sind wertvolle Zwischenprodukte für die Herstellung von Metallkomplexfarbstoffen, insbesondere von Reaktivfarbstoffen auf Basis von Formazanen (siehe z.B. EP-A-592 980).

Die folgenden Beispiele sollen die Erfindung näher erläutern.

### Beispiele

### Beispiel 1

a) Zu 160 g Natriumhydroxid in 1,5 l Wasser wurden unter Kühlung bei 20 bis 40°C 232 g 3-Amino-4-hydroxy-5-nitrobenzolsulfonsäure eingetragen. Die Reaktionsmischung wurde auf 0 bis 10°C abgekühlt, dann wurden innerhalb von 8 Stunden 182 g Phosgen eingeleitet, wobei der pH-Wert mit 150 g 50 gew.-%iger Natronlauge zwischen 7,5 und 8,5 gehalten wurde. Es wurde eine Stunde nachgerührt und anschließend überschüssiges Phosgen mit Stickstoff ausgeblasen. Der pH-Wert wurde mit 200 g konz. Salzsäure auf 7 gestellt. Zur entstandenen 7-Nitrobenzoxazol-2-on-5-sulfonsäure (Natriumsalz) wurden 67 g Ethanolamin gegeben und der pH-Wert mit 164 g konz. Salzsäure auf 7 gestellt. Es wurde auf 60°C erwärmt und 6,5 Stunden bei dieser Temperatur gerührt. Nach vollständigem Umsatz (DC-Kontrolle) wurde mit konz. Salzsäure auf einen pH-Wert von 1,5 gestellt und das ausgefallene Produkt abgesaugt. Nach dem Trocknen bei 70 bis 80°C unter vermindertem Druck erhielt man 289 g der Verbindung der Formel 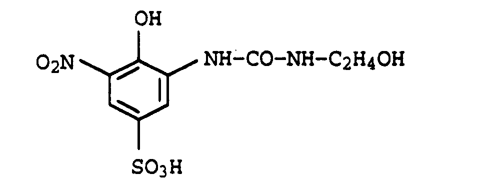
   - ¹H-NMR [D⁶-DMSO]: δ =: 3,2, 3,4 (jeweils CH₂)
   4,7 (OH),
   7,7, 8,2 (Aromaten-H)
   8,1, 8,9 (NH)
b) 280 g der unter a) erhaltenen Nitroverbindung wurden in 3 l Wasser gelöst. Man gab 5 g Palladiumkatalysator (10 gew.-%ig auf Kohle) zu und hydrierte bei 35 bis 40°C. Nach beendeter Wasserstoffaufnahme wurde Vom Katalysator abfiltriert und die Mutterlauge eingedampft. Es verblieben 250 g der Verbindung der Formel 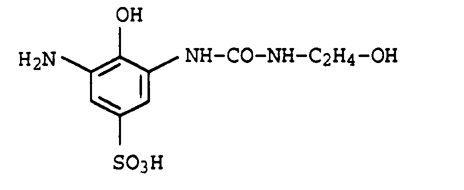
   - ¹H-NMR [D⁶-DMSO]: δ =: 3,2, 3,4 (jeweils CH₂)
   4,7 (OH),
   7,35, 7,50 (Aromaten-H)
   7,10, 8,70 (NH).

Analog Beispiel 1a) können die in Tabelle 1 aufgeführten Verbindungen erhalten werden.

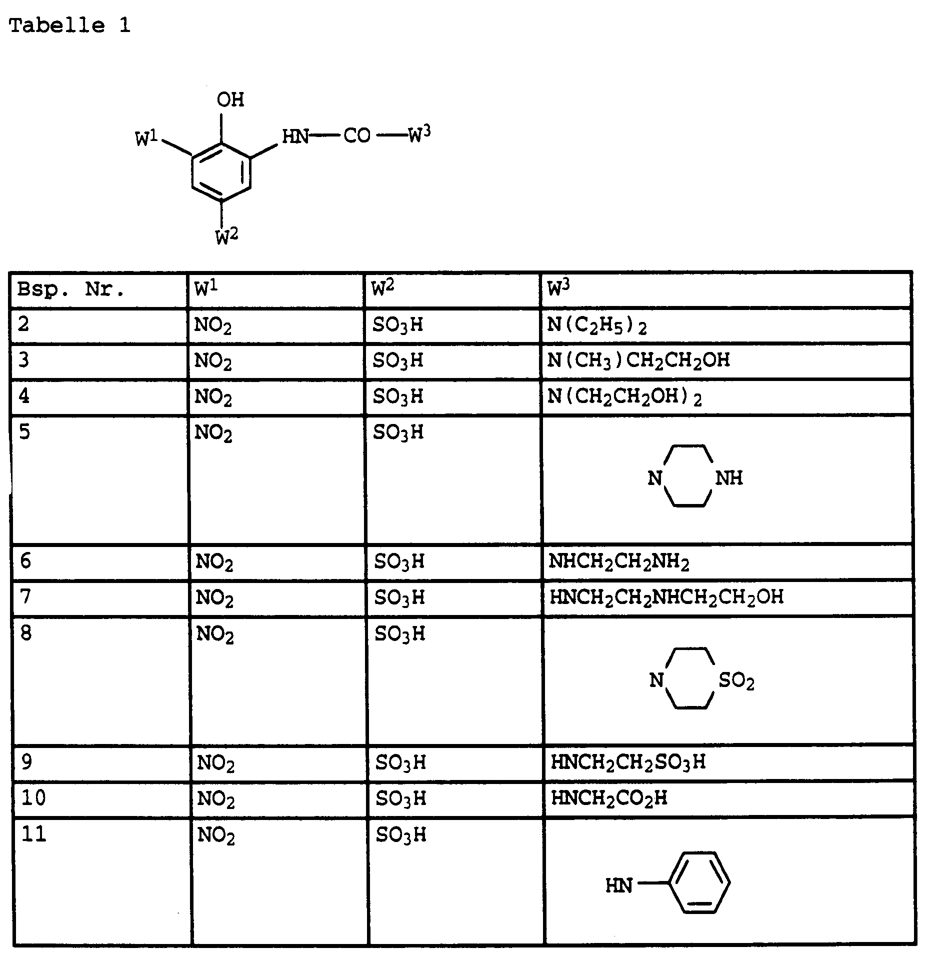

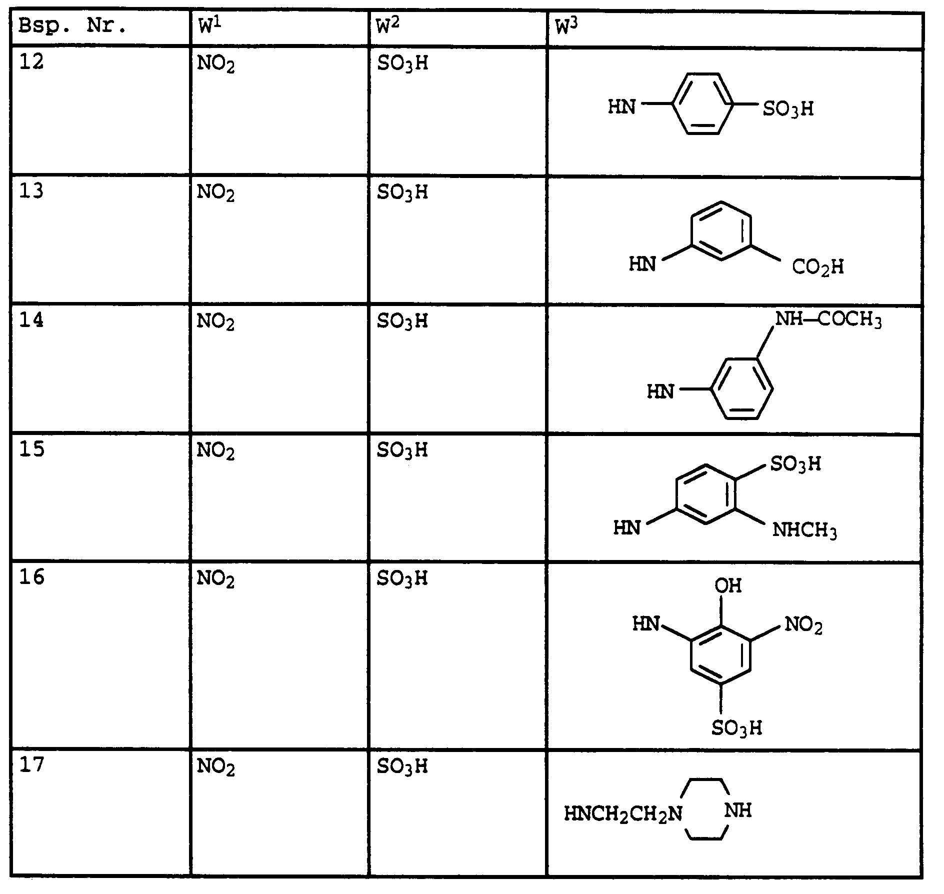

Durch katalytische Hydrierung der obengenannten Nitroverbindungen analog Beispiel 1b können die entsprechenden Aminoverbindungen (W¹ = NH₂) erhalten werden.

### Beispiel 18

a) Man verfuhr analog Beispiel 1a, verwendete jedoch anstelle von 3-Amino-4-hydroxy-5-nitrobenzolsulfonsäure 232 g der isomeren 2-Hydroxy-3-amino-5-nitrobenzolsulfonsäure. Man erhielt nach dem Trocknen bei 70 bis 80°C unter vermindertem Druck 365 g der Verbindung der Formel 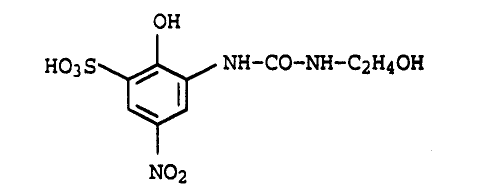
b) Man reduzierte die unter a) erhaltene Nitroverbindung gemäß Beispiel 1b. Man erhielt nach dem Trocknen bei 30 bis 40°C 295 g der Verbindung der Formel

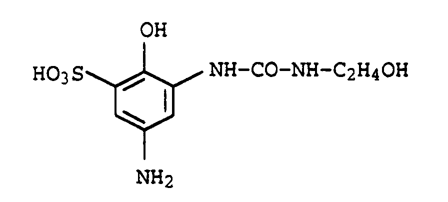

Analog Beispiel 18a) können, unter Verwendung entsprechend substituierter o-Amino-hydroxy-benzolderivate, die in der folgenden Tabelle 2 aufgeführten Verbindungen erhalten werden.

Durch katalytische Hydrierung der in Tabelle 2 aufgeführten Nitroverbindungen analog Beispiel 1b) können die entsprechenden Aminoverbindungen erhalten werden.

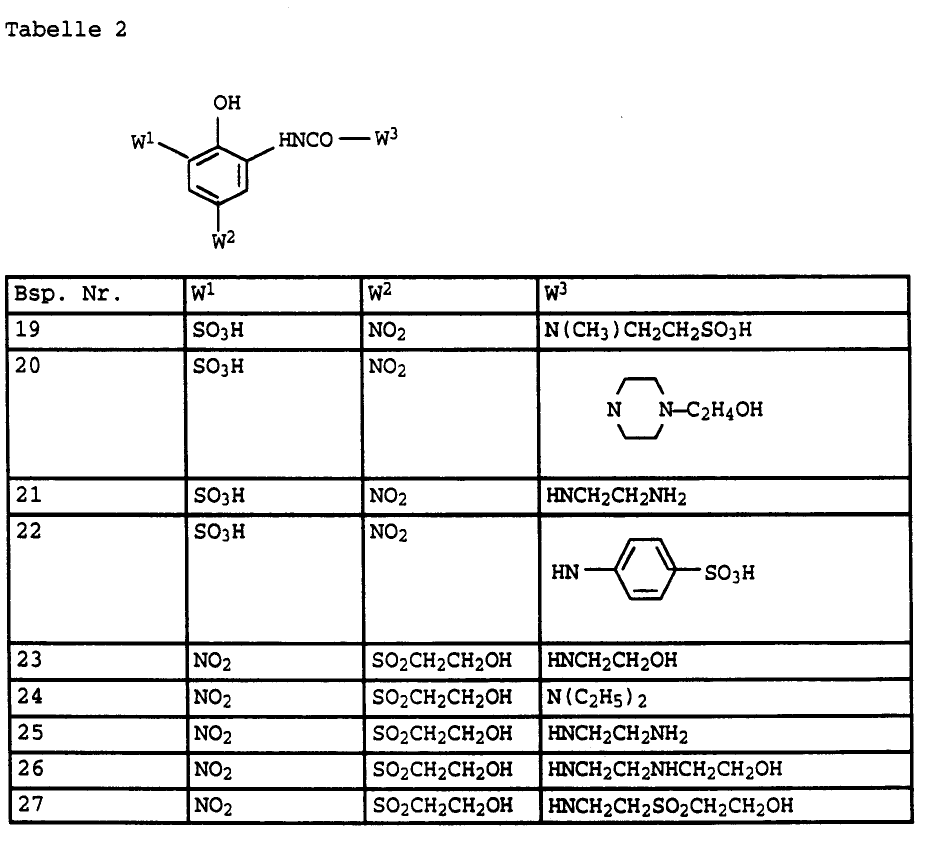

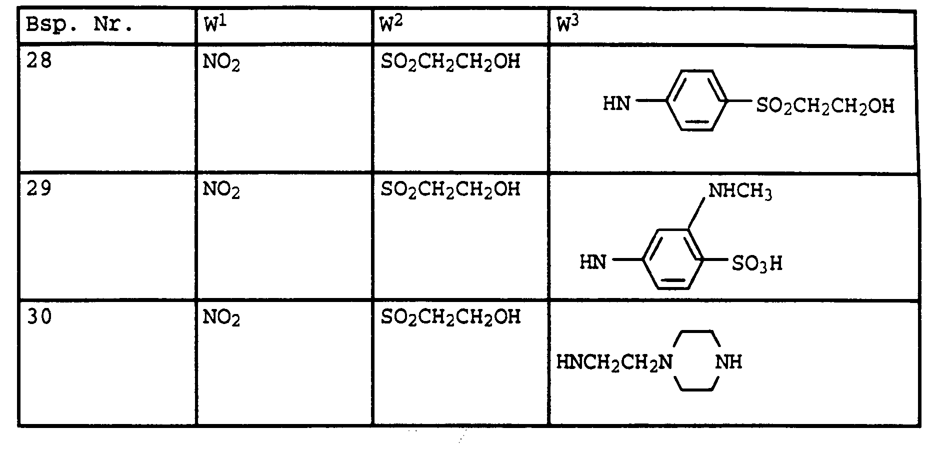

### Beispiel 31

a) Man verfuhr analog Beispiel 1a), erhöhte jedoch nach dem Ausblasen von Phosgen dem pH-Wert auf 8,0 bis 8,5 und fügte 148 g Hydroxymethansulfonsäure zu. Dann rührte man 4 Stunden bei 60 bis 80°C bis zur vollständigen Alkylierung nach (DC-Reaktionskontrolle), gab danach 153 g 2-Aminoethyl-2'-hydroxyethylsulfon zu und fuhr fort wie in Beispiel 1a) beschrieben. Man erhielt 383 g der Verbindung der Formel 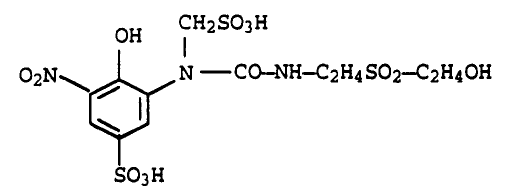
   - ¹H-NMR [D⁶-DMSO]: δ =: 3,10, 3,20, 3,30, 3,40, 3,80 (jeweils CH₂), 4,20 (OH)
   6,50 (NH)
   7,50, 8,00 (Aromaten-H).
b) 306 g der unter a) erhaltenen Nitroverbindung wurden in 3 l Wasser gelöst. Man gab 5 g Palladiumkatalysator (10 gew.-%ig auf Kohle) zu und hydrierte bei 35 bis 40°C. Nach beendeter Wasserstoffaufnahme wurde vom Katalysator abfiltriert und die Mutterlauge eingedampft. Es verbleiben 250 g der Verbindung der Formel 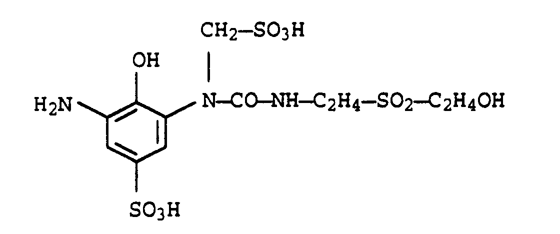
   - ¹H-NMR [D⁶-DMSO]: δ =: 2,40, 2,90, 3,15, 3,40, 3,75 (jeweils CH₂)
   4,50 (OH, NH₂), 6,40 (NH)
   6,60, 6,90 (Aromaten-H).
c) 280 g des unter b) erhaltenen Harnstoffs wurden unter Eiskühlung bei 10 bis 15°C in 1120 g Chlorsulfonsäure eingetragen und ca. 12 Stunden bei Raumtemperatur gerührt. Nach vollständigem Umsatz (DC-Kontrolle) wurde auf 4 l Eis gefällt, das ausgefallene Produkt abgesaugt und mit Wasser neutralgewaschen. Nach dem Trocknen unter vermindertem Druck bei 30 bis 40°C verblieben 302 g der Verbindung der Formel 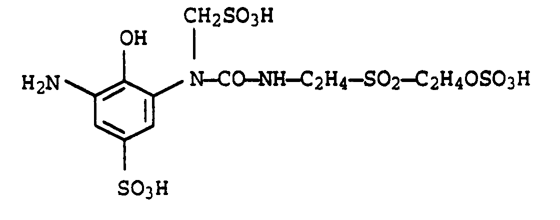 die ohne weitere Reinigung zu Farbstoffsynthesen verwendet werden kann.

Verfährt man analog Beispiel 31, fügt jedoch anstelle von Hydroxymethansulfonsäure 1,1 mol eines Alkylierungsmittels, wie Dimethylsulfat, p-Toluolsulfonsäuremethylester, Ethyliodid, Ethylenoxid, Butansulton-(1,4) oder Allylbromid, zu, rührt 4 Stunden bei 60 bis 80°C bis zum vollständigen Umsatz (DC-Reaktionskontrolle), gibt dann 1,1 mol eines Alkyl- oder Arylamins zu und fährt fort wie in Beispiel 31 beschrieben, so erhält man unter Verwendung entsprechender o-Amino-hydroxy-benzolderivate die in folgender Tabelle aufgeführten Harnstoffe.

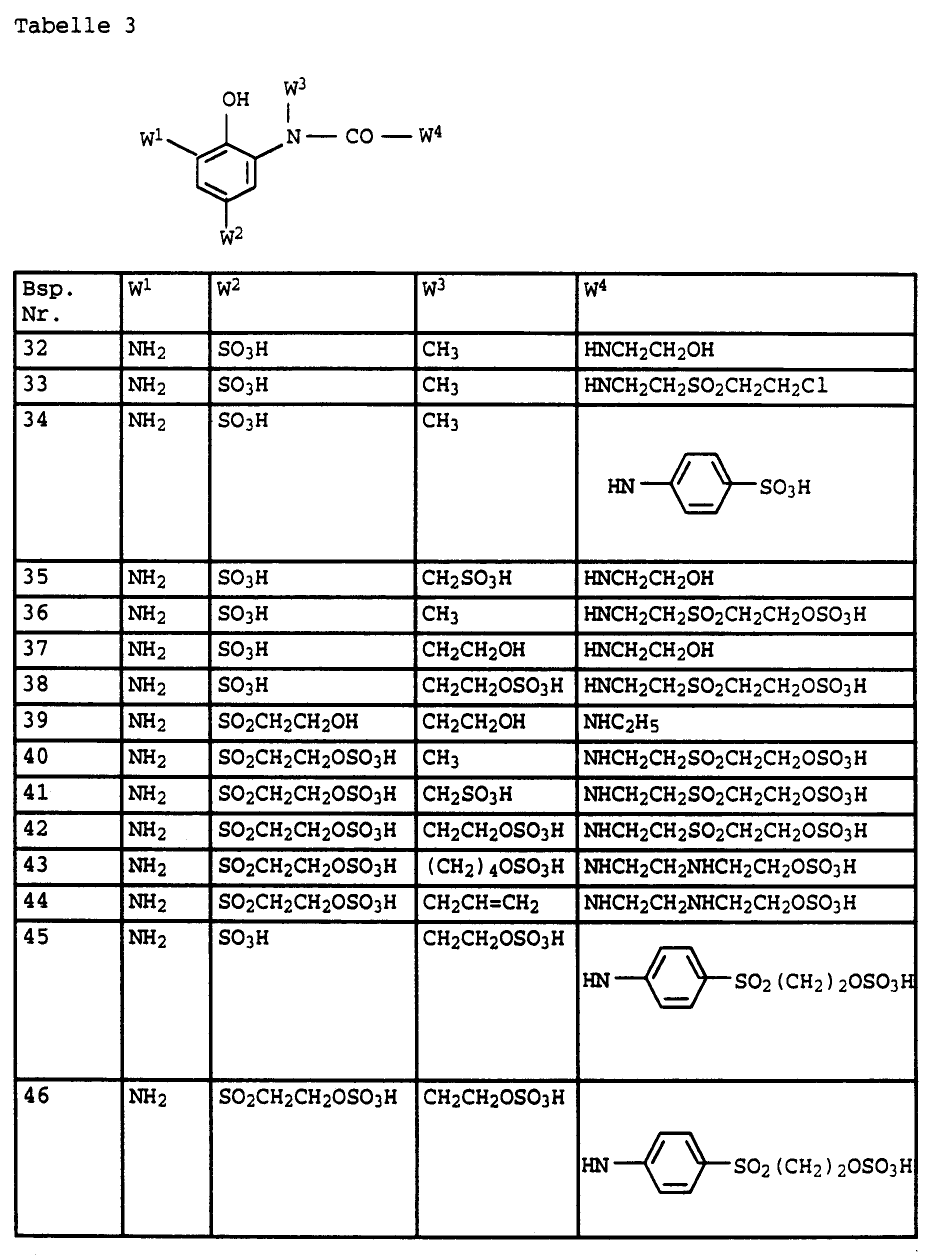

### Beispiel 47

Man verfuhr analog Beispiel 1a) setzte jedoch anstelle von Ethanolamin 30 g (0,5 mol) Ethylendiamin zu. Man erhielt nach dem Ausfällen, Filtrieren und Trocknen bei 70 bis 80°C unter vermindertem Druck 310 g der Verbindung der Formel 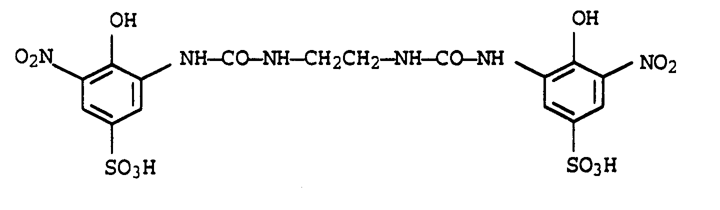
- ¹H-NMR [D⁶-DMSO]: δ =: 3,4 (CH₂)
7,7, 8,2 (Aromaten-H)
7,7, 8,6 (NH).

Analog Beispiel 47 können die in Tabelle 4 aufgeführten Verbindungen erhalten werden.

Durch katalytische Hydrierung über Palladium/Kohle können die entsprechenden Aminoverbindungen (W¹ = NH₂) erhalten werden.

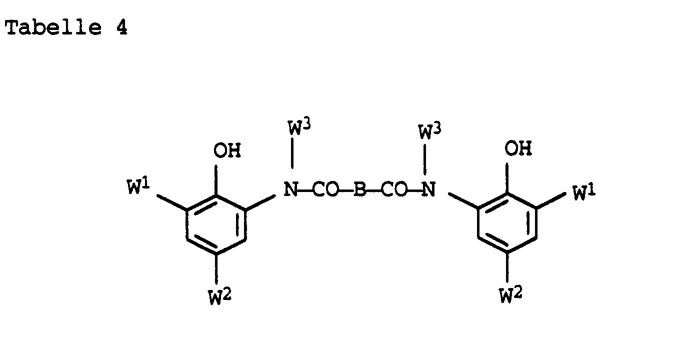

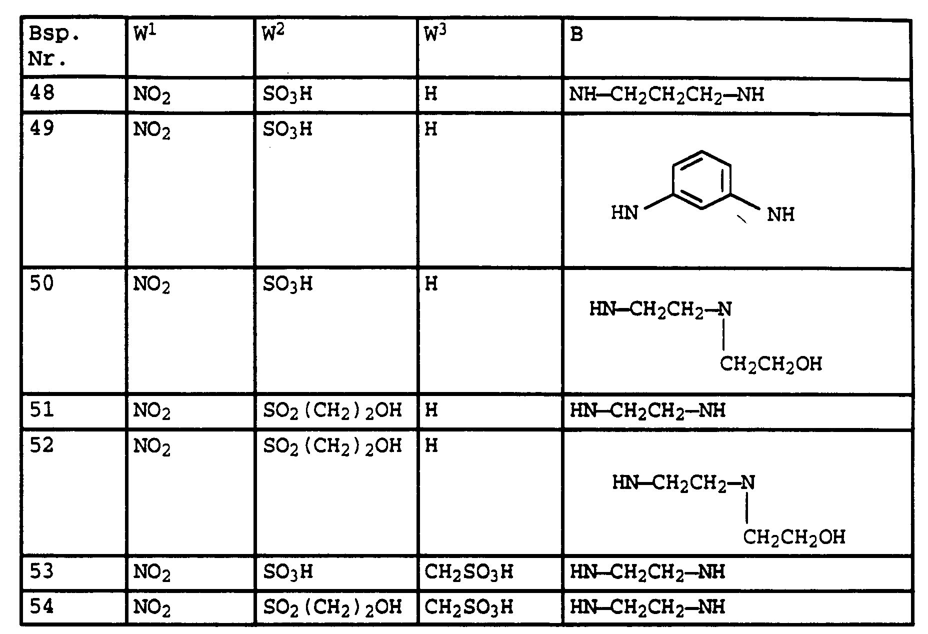

## Patentansprüche

1. Harnstoffe der Formel I 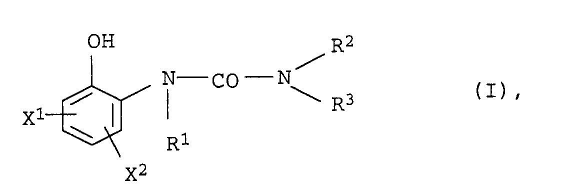 in der
X¹ Nitro oder Amino,
X² Nitro, Amino, Hydroxysulfonyl, Sulfamoyl, C₁-C₄-Mono- oder Dialkylsulfamoyl oder einen Rest der Formel -S(O)ₙ-Y, worin n für 0 oder 2 und Y für Vinyl oder einen Rest der Formel C₂H₄-Q stehen, in dem Q die Bedeutung von Hydroxy oder einer unter alkalischen Reaktionsbedingungen abspaltbaren Gruppe besitzt,
R¹ Wasserstoff, C₁-C₆-Alkyl, das gegebenenfalls substituiert ist und durch 1 oder 2 Sauerstoffatome in Etherfunktion oder Imino- oder C₁-C₄-Alkyliminogruppen unterbrochen sein kann, oder C₃-C₄-Alkenyl,
R² und R³ unabhängig voneinander jeweils C₁-C₆-Alkyl, das gegebenenfalls substituiert ist und durch 1 oder 2 Sauerstoffatome in Etherfunktion oder Imino- oder C₁-C₄-Alkyliminogruppen unterbrochen sein kann, oder gegebenenfalls substituiertes Phenyl oder R² auch Wasserstoff oder einen Rest der Formel 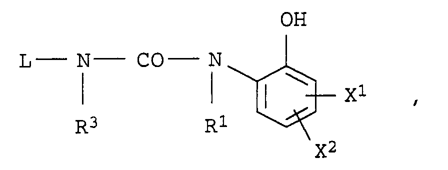 worin L für C₂-C₈-Alkylen oder Phenylen steht und R¹, R³, X¹ und X² jeweils die obengenannte Bedeutung besitzen, oder R² und R³ zusammen auch einen Rest der Formel (-C₂H₄)₂N-R¹, worin R¹ die obengenannte Bedeutung besitzt, bedeuten,
mit der Maßgabe, daß
a) Verbindungen der Formel 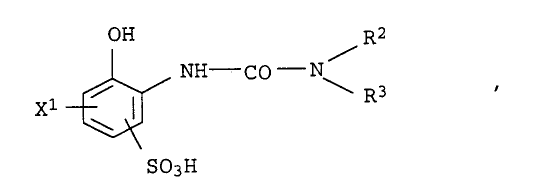 in der X¹ Nitro oder Amino und einer oder beide Reste R² und R³ C₁-C₆-Alkyl, das durch den Rest -S(O)ₙ-Y, in dem n und Y jeweils die obengenannte Bedeutung besitzen, substituiert und gegebenenfalls durch 1 oder 2 Sauerstoffe in Etherfunktion oder Imino- oder C₁-C₄-Alkyliminogruppen unterbrochen ist, bedeuten,
b) Verbindungen der Formel 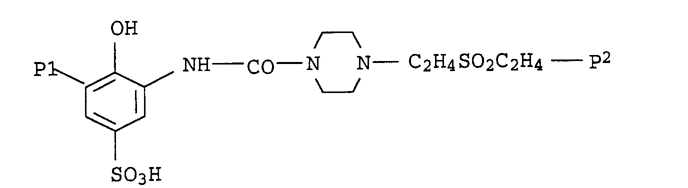
| P¹ | P² |
|---|---|
| NO₂ | OH |
| NH₂ | OH |
| NH₂ | OSO₃H |
und
c) Die Verbindung der Formel 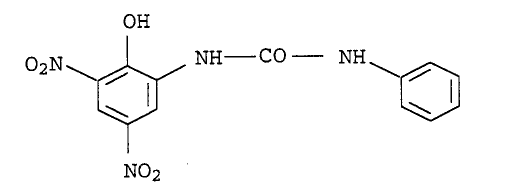 ausgenommen sind.

2. Harnstoffe nach Anspruch 1, dadurch gekennzeichnet, daß
X² Nitro, Amino, Hydroxysulfonyl oder einen Rest der Formel -SO₂-Y bedeutet, worin Y die in Anspruch 1 genannte Bedeutung besitzt.

3. Harnstoffe nach Anspruch 1, dadurch gekennzeichnet, daß
R¹ Wasserstoff oder gegebenenfalls durch Hydroxysulfonyl, Hydroxy oder Sulfato substituiertes C₁-C₆-Alkyl bedeutet.

4. Harnstoffe nach Anspruch 1, dadurch gekennzeichnet, daß
R² Wasserstoff oder C₁-C₆-Alkyl und
R³ C₁-C₆-Alkyl, das gegebenenfalls durch Amino, Hydroxy, Hydroxysulfonyl, Sulfato oder einen Rest der Formel -SO₂-Y, worin Y die in Anspruch 1 genannte Bedeutung besitzt, substituiert ist und durch ein Sauerstoffatom in Etherfunktion oder eine Iminogruppe unterbrochen ist, oder Phenyl, das gegebenenfalls durch Amino, C₁-C₄-Monoalkylamino, C₁-C₄-Alkanoylamino, Benzoylamino, Carboxyl, Hydroxysulfonyl oder einen Rest der Formel -SO₂-Y, worin Y die in Anspruch 1 genannte Bedeutung besitzt, substituiert ist, oder R² und R³ zusammen einen Rest der Formel (-C₂H₄)₂N-R¹, worin R¹ die in Anspruch 1 genannte Bedeutung besitzt, bedeuten.

5. Harnstoffe nach Anspruch 1, dadurch gekennzeichnet, daß
L C₂-C₄-Alkylen oder Phenylen bedeutet.

6. Verfahren zur Herstellung von Harnstoffen gemäß Anspruch 1, dadurch gekennzeichnet, daß man ein Benzoxazolon der Formel II 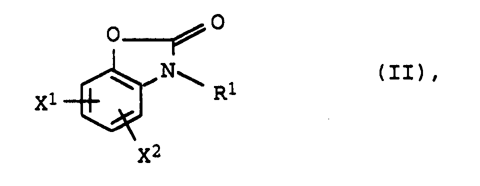 in der X¹, X² und R¹ jeweils die in Anspruch 1 genannte Bedeutung besitzen, mit einem Amin der Formel III 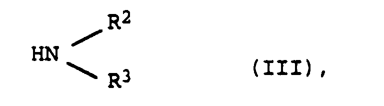 in der R² und R³ jeweils der in Anspruch 1 genannte Bedeutung besitzen, umsetzt.

## Claims

1. Ureas of the formula I 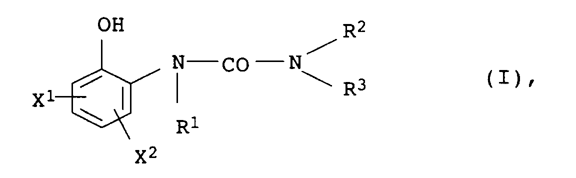 where
X¹ is nitro or amino,
X² is nitro, amino, hydroxysulfonyl, sulfamoyl, mono- or di(C₁-C₄-alkyl)sulfamoyl or a radical of the formula -S(O)ₙ-Y, where n is 0 or 2 and Y is vinyl or a radical of the formula C₂H₄-Q, where Q is hydroxyl or contains an alkali-detachable group,
R¹ is hydrogen, C₁-C₆-alkyl with or without substitution and with or without interruption by 1 or 2 oxygen atoms in ether function or by imino or C₁-C₄-alkylimino groups, or C₃-C₄-alkenyl,
R² and R³ are independently of each other C₁-C₆-alkyl with or without substitution and with or without interruption by 1 or 2 oxygen atoms in ether function or imino or C₁-C₄-alkylimino groups, or substituted or unsubstituted phenyl, or R² is hydrogen or a radical of the formula 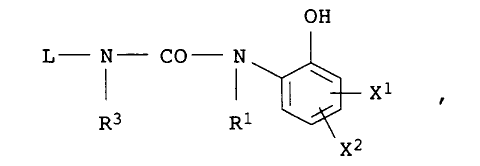 where L is C₂-C₈-alkylene or phenylene and R¹, R³, X¹ and X² are each as defined above, or R² and R³ are together a radical of the formula (-C₂H₄)₂N-R¹, where R¹ is as defined above,
with the proviso that
a) compounds of the formula 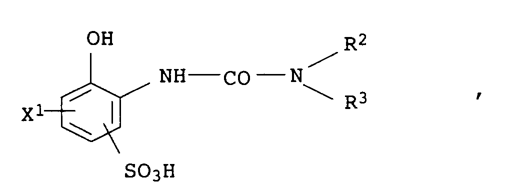 where X¹ is nitro or amino and R² and R³ are either or both C₁-C₆-alkyl with or without substitution by the radical -S(O)ₙ-Y, where n and Y are each as defined above, and with or without interruption by 1 or 2 oxygen atoms in ether function or by imino or C₁-C₄-alkylimino groups,
b) compounds of the formula 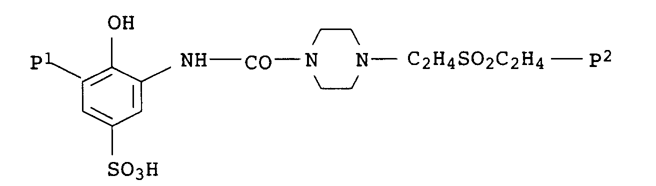
| P¹ | P² |
|---|---|
| NO₂ | OH |
| NH₂ | OH |
| NH₂ | OSO₃H |
and
c) the compound of the formula 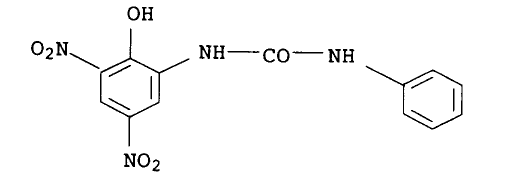 are excluded.

2. Ureas as claimed in claim 1, wherein
X² is nitro, amino, hydroxysulfonyl or a radical of the formula -SO₂-Y, where Y is as defined in claim 1.

3. Ureas as claimed in claim 1, wherein
R¹ is hydrogen or unsubstituted or hydroxysulfonyl-, hydroxyl- or sulfato-substituted C₁-C₆-alkyl.

4. Ureas as claimed in claim 1, wherein
R² is hydrogen or C₁-C₆-alkyl, and
R³ is C₁-C₆-alkyl with or without substitution by amino, hydroxyl, hydroxysulfonyl, sulfato or a radical of the formula -SO₂-Y, where Y is as defined in claim 1, and with or without interruption by an oxygen atom in ether funtion or by an imino group, or is phenyl with or without substitution by amino, mono-C₁-C₄-alkylamino, C₁-C₄-alkanoylamino, benzoylamino, carboxyl, hydroxysulfonyl or a radical of the formula -SO₂-Y, where Y is as defined in claim 1, or R² and R³ are together a radical of the formula (-C₂H₄)₂N-R¹, where R¹ is as defined in claim 1.

5. Ureas as claimed in claim 1, wherein
L is C₂-C₄-alkylene or phenylene.

6. A process for preparing ureas as claimed in claim 1, which comprises reacting a benzoxazolone of the formula II 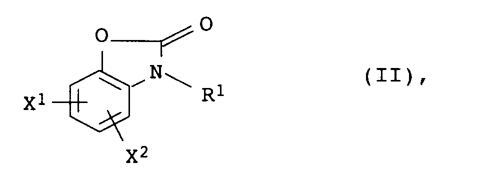 where X¹, X² and R¹ are each as defined in claim 1, with an amine of the formula III 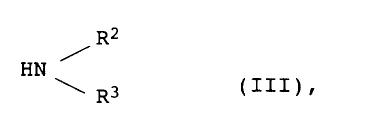 where R² and R³ are each as defined in claim 1.

## Revendications

1. Composés de l'urée de formule I 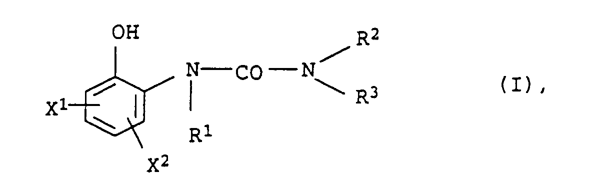 dans laquelle
X¹ est mis pour un groupement nitro ou amino,
X² est mis pour un groupement nitro, amino, hydroxysulfonyle, sulfamoyle, mono- ou dialkyl( en C₁-C₄)sulfamoyle ou un reste de formule -S(O)ₙ-Y, où n est mis pour 0 ou 2 et Y pour un groupement vinyle ou un reste de formule C₂H₄-Q, dans laquelle Q représente un groupement hydroxy ou un groupement libérable dans des conditions réactionnelles alcalines,
R¹ est mis pour un atome d'hydrogène, un groupement alkyle en C₁-C₆, qui est éventuellement substitué et peut être interrompu par 1 ou 2 atomes d'oxygène en fonction éther ou par des groupements imino ou alkyl( en C₁-C₄)imino, ou bien pour un groupement alcényle en C₃-C₄,
R² et R³ sont mis chacun, indépendamment l'un de l'autre, pour un groupement alkyle en C₁-C₆, éventuellement substitué et qui peut être interrompu par 1 ou 2 atomes d'oxygène en fonction éther ou par des groupements imino ou alkyl( en C₁-C₄)imino, ou pour un groupement phényle éventuellement substitué ou bien R² peut aussi représenter un atome d'hydrogène ou un reste de formule 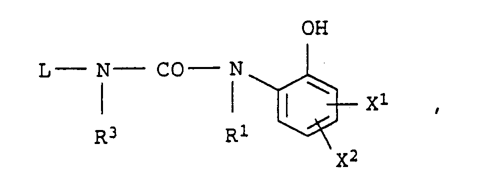 où L est mis pour un groupement alkylène en C₂-C₈ ou phénylène, et R¹, R³, X¹ et X² prennent chacun la signification susmentionnée, ou bien R² et R³ peuvent représenter ensemble un reste de formule (-C₂H₄)₂N-R¹, où R¹ prend la signification susmentionnée,
étant spécifié que
a) les composés de formule 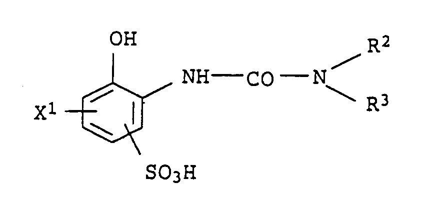 dans laquelle X¹ est mis pour un groupement nitro ou amino et l'un ou les deux restes R² et R³ sont mis pour un groupement alkyle en C₁-C₆, substitué par un reste -S(O)ₙ-Y, dans lequel n et Y prennent chacun la signification susmentionnée, et éventuellement interrompu par un ou deux atomes d'oxygène en fonction éther ou par des groupements imino ou alkyl(en C₁-C₄)imino,
b) les composés de formule 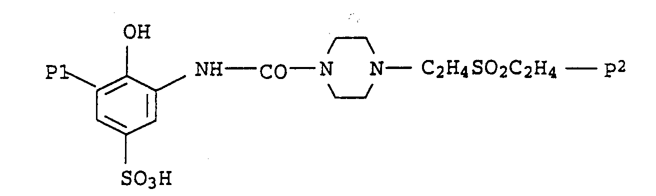
| P¹ | P² |
|---|---|
| NO₂ | OH |
| NH₂ | OH |
| NH₂ | OSO₃H |
et
c) le composé de formule 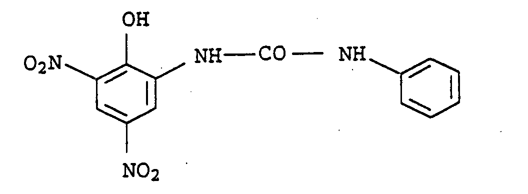 sont exclus.

2. Composés de l'urée selon la revendication 1, caractérisés en ce que
X² est mis pour un groupement nitro, amino, hydroxysulfonyle ou un reste de formule -SO₂-Y, où Y prend la signification mentionnée dans la revendication 1.

3. Composés de l'urée selon la revendication 1, caractérisés en ce que
R¹ est mis pour un atome d'hydrogène ou pour un groupement alkyle en C₁-C₆ éventuellement substitué par un groupement hydroxysulfonyle, hydroxy ou sulfato.

4. Composés de l'urée selon la revendication 1, caractérisés en ce que
R² est mis pour un atome d'hydrogène ou un groupement alkyle en C₁-C₆ et
R³ est mis pour un groupement alkyle en C₁-C₆, éventuellement substitué par un groupement amino, hydroxy, hydroxysulfonyle, sulfato ou un reste de formule -SO₂Y, où Y prend la signification mentionnée dans la revendication 1, et interrompu par un atome d'oxygène en fonction éther ou par un groupement imino, ou pour un groupement phényle, éventuellement substitué par un groupement amino, monoalkyl(en C₁-C₄)amino, alcanoyl(en C₁-C₄)amino, benzoylamino, carboxyle, hydroxysulfonyle ou un reste de formule -SO₂Y, où Y prend la signification mentionnée dans la revendication 1, ou bien R² et R³ représentent ensemble un reste de formule(-C₂H₄)₂N-R¹, où R¹ prend la signification mentionnée dans la revendication 1.

5. Composés de l'urée selon la revendication 1, caractérisés en ce que
L est mis pour un groupement alkylène en C₂-C₄ ou phénylène.

6. Procédé de préparation de composés de l'urée selon la revendication 1, caractérisé en ce que l'on fait réagir une benzoxazolone de formule II 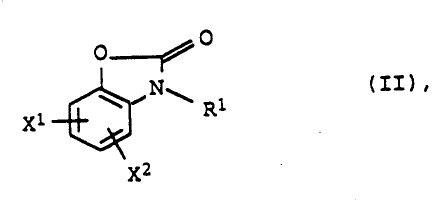 dans laquelle X¹, X² et R¹ prennent chacun la signification mentionnée dans la revendication 1, avec une amine de formule III 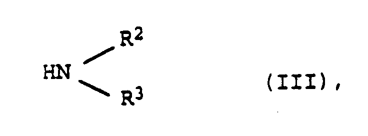 dans laquelle R² et R³ prennent chacun la signification mentionnée dans la revendication 1.
